# EUROPÄISCHE PATENTSCHRIFT

(11) **EP 4 258 992 B1**
(45) Veröffentlichungstag und Bekanntmachung des Hinweises auf die Patenterteilung: **30.10.2024**
(21) Anmeldenummer: 21830622.3
(22) Anmeldetag: 01.12.2021
(51) Int. Cl.: A61B 5/1455, A61B 5/0205, A61M 16/00

(54) **VERFAHREN ZUR MESSUNG DER REAKTION EINES PATIENTEN AUF EIN HYPOXIETRAINING**
A METHOD OF MEASURING THE RESPONSE OF A PATIENT TO HYPOXIC TRAINING
PROCÉDÉ DE MESURE DE LA RÉPONSE D'UN PATIENT À UN ENTRAÎNEMENT HYPOXIQUE

(30) Priorität: 11.12.2020 DE 102020215742
(43) Veröffentlichungstag der Anmeldung: 18.10.2023
(73) Patentinhaber: GOYTIA, Rainer, 73663 Berglen (DE)
(72) Erfinder: GOYTIA, Rainer, 73663 Berglen (DE)
(74) Vertreter: Kohler Schmid Möbus Patentanwälte
(86) Internationale Anmeldenummer: PCT/EP2021/083816
(87) Internationale Veröffentlichungsnummer: WO 2022/122511

(56) Entgegenhaltungen:
- EP-A1- 1 721 629
- DE-A1- 102012 010 806
- US-A1- 2016 095 994
- "Intermittent Hypoxia: From Molecular Mechanisms to Clinical Applications", 1 January 2011, article BASSOVITCH OLEG . ET AL: "Equipment and regimes for intermittent hypoxia therapy", pages: 589 - 601, XP55901732
- "Intermittent Hypoxia: From Molecular Mechanisms to Clinical Applications", 1 January 2011, article BASSOVITCH OLEG . ET AL: "Equipment and regimes for intermittent hypoxia therapy", pages: 589 - 601, XP055901732
- K. P. SAUSEN ET AL: "A Closed-Loop Reduced Oxygen Breathing Device for Inducing Hypoxia In Humans", AVIATION, SPACE, AND ENVIRONMENTAL MEDICINE, vol. 74, 1 January 2003 (2003-01-01), pages 1190 - 1197, XP055436960
- BALIOZ N V ET AL: "Individual typological features in the EEG of athletes after acute hypoxic treatment", HUMAN PHYSIOLOGY, KLUWER ACADEMIC PUBLISHERS-PLENUM PUBLISHERS, NE, vol. 38, no. 5, 10 October 2012 (2012-10-10), pages 470 - 477, XP035123047, ISSN: 1608-3164, DOI: 10.1134/S0362119712050027

## Beschreibung

### Hintergrund der Erfindung

Die Erfindung betrifft ein Verfahren zur Messung eines Hypoxic-Session-Index als Maß der Reaktion eines Patienten auf ein Hypoxietraining oder eine Hypoxietherapie-Sitzung, unter Messung einer Kenngröße, die die periphere Sauerstoffsättigung des Bluts des Patienten und/oder die Reaktion des Pulses kennzeichnet. Weiterhin wird eine Vorrichtung zur Durchführung des Verfahrens offenbart.

Aus dem Stand der Technik sind Verfahren zur Durchführung einer Hypoxietherapie-Sitzung bekannt:
Die DE 10 2012 010 806 A1 offenbart eine Hypoxietherapie-Sitzung, bei dem die Werte der peripheren Sauerstoffsättigung und des Pulses eines Patienten an eine Überwachungseinrichtung gesendet werden, während dem Patienten eine hypoxische Gasmischung im Wechsel mit einer normoxischen oder hperoxischen Gasmischung zugeführt wird. Die Zuführung der hypoxischen Gasmischung und der normoxischen oder hyperoxischen Gasmischung erfolgt dabei jeweils über einen vorher festgelegten Zeitraum.

Die US 2016/095994 A1 offenbart ein Verfahren zur Messung eines Hypoxic-Training-Index (HTI), wobei ein Patient in einer Hypoxiephase über einen vorgegebenen Hypoxie-Zeitraum mit einem hypoxischen Gasgemisch versorgt wird, der Patient in einer Reoxygenierungsphase über einen vorgegebenen Hyperoxie-Zeitraum und in einem sich daran anschließenden, vorbestimmten Abschlusszeitraum mit einem normoxischen Gasgemisch versorgt wird, die Sauerstoffsättigung über die Zeit in der Hypoxiephase und der Reoxygenierungsphase in Form einer Datenkurve aufgetragen wird und aus aus einer Abweichung der Datenkurve von einem Referenzwert von 90% Sauerstoffsättigung ein Hypoxic-Training-Index bestimmt wird.

Die bekannten Verfahren bieten eine vergleichsweise geringe Unterstützung bei der Auswertung der in dem Hypoxietraining gewonnenen Daten.

### Aufgabe der Erfindung

Es ist daher Aufgabe der vorliegenden Erfindung, ein Verfahren anzugeben, bei welchem die im Hypoxietraining gewonnenen Daten in Form quantitativer Ergebnisse ausgewertet werden.

### Kurze Beschreibung der Erfindung

Diese Aufgabe wird erfindungsgemäß durch eine Verfahren nach Anspruch 1 gelöst. Vorteilhafte Ausgestaltungen ergeben sich aus den jeweilig rückbezogenen Unteransprüchen.

Das erfindungsgemäße Verfahren weist die folgenden Schritte auf:
I. Versorgen des Patienten mit einem normoxischen Gasgemisch in einer Anfangsphase über einen vorgegebenen ersten Zeitraum;
II. Bestimmen des Mittelwerts der Kenngröße in der Anfangsphase;
III. Versorgen des Patienten mit einem hypoxischen Gasgemisch in einer Hypoxiephase über einen vorgegebenen Hypoxie-Zeitraum;
IV. Versorgen des Patienten mit dem normoxischen oder hyperoxischen Gasgemisch in einer Reoxygenierungsphase über einen vorgegebenen Hyperoxie-Zeitraum und in einem sich daran anschließenden, vorbestimmten Abschlusszeitraum;
V. Auftragen der Kenngröße über die Zeit in der Anfangsphase, der Hypoxiephase und der Reoxygenierungsphase in Form einer Datenkurve;
VI. Bestimmen des Hypoxic-Session-Index aus einer Abweichung der Datenkurve von einer vorbestimmten Referenzkurve, wobei die Referenzkurve eine Datenkurve ist, die nach den Schritten I bis V bei einer Referenzperson bestimmt wird, oder eine Datenkurve ist, die durch eine Mittelung von Datenkurven mehrerer Referenzpersonen berechnet wird, wobei die Datenkurven nach den Schritten I bis V bei den Referenzpersonen bestimmt wird.

Die Abweichung zwischen der Datenkurve und der Referenzkurve ermöglicht eine quantitative Bewertung der Messwerte der Sauerstoffsättigung. Die Kenngröße ist insbesondere die Sauerstoffsättigung oder der Sauerstoffpartialdruck des im Blut des Patienten gelösten Sauerstoffs, deren Zusammenhang durch die Sauerstoffbindungskurve mit einem sigmoidalen Verlauf dargestellt wird. Die Abweichung wird insbesondere durch einen Abstand zwischen der Datenkurve und der Referenzkurve oder durch einen Vergleich von Flächen, die durch die Datenkurve oder die Referenzkurve begrenzt werden, bestimmt.

Ein Hypoxie-Sicherheitswert, der insbesondere den kleinsten Wert angibt, bis zu dem die Kenngröße in der Hypoxie-Phase abfallen darf, liegt insbesondere zwischen 70% und 88% des Mittelwerts der Kenngröße in der Anfangsphase, insbesondere bei 80% des Mittelwerts der Kenngröße in der Anfangsphase.

In der Anfangsphase ist der Patient insbesondere körperlich nicht belastet und atmet Raumluft. Der Patient befindet sich in einer Ruheposition, insbesondere auf einer Liege liegend. In der Anfangsphase wird insbesondere die Vorrichtung zur Durchführung der Messung des Hypoxic-Session-Index kalibriert.Die Anfangsphase beträgt insbesondere zwischen 1 Minute und 5 Minuten. In der Anfangsphase wird insbesondere in einem Zeitraum zwischen 20 Sekunden und 120 Sekunden, insbesondere 30 Sekunden, vor Beginn der ersten Hypoxiephase in jeder Sekunde die Kenngröße, zum Beispiel der Sauerstoffpartialdruck, gemessen und festgehalten. Nach dem Ende der Anfangsphase wird der Mittelwert der Kenngröße zur Bestimmung einer Baseline der Kenngröße während des Hypoxietrainings berechnet. Der vorgegebene Hypoxie-Zeitraum und/oder Hyperoxie-Zeitraum beträgt insbesondere zwischen 1 Minute und 15 Minuten. Die Bestimmung des Mittelwerts der Kenngröße in der Anfangsphase gemäß Schritt II erfolgt insbesondere vor der Hypoxiephase gemäß Schritt III, vorzugsweise während der Anfangsphase oder zum Endzeitpunkt der Anfangsphase. Bei einer alternativen Ausgestaltung des Verfahrens sind die Hypoxiephase gemäß Schritt III und die Hyperoxiephase gemäß Schritt IV vertauscht.

### Bevorzugte Ausgestaltungen der Erfindung

Eine Referenzperson ist insbesondere eine Person, die als gesund eingestufte Messwerte der Kenngröße aufweist. Die Referenzkurve kann auch durch eine Mittelung der Messwerte der Kenngröße der Referenzpersonen zum jeweiligen gleichen Zeitpunkt des Verfahrens bei mehreren Referenzpersonen ermittelt werden. Gegebenenfalls kann eine Unterteilung der Referenzpersonen hinsichtlich ihrer körperlichen Merkmale erfolgen.

Bei einigen Ausgestaltungen des Verfahrens wird der Hypoxic-Session-Index als Liste von Indices dargestellt, insbesondere von Indices, die im folgenden Text dargestellt sind.

Vorteilhaft ist das Verfahren durch das Festlegen eines Hypoxic-Cycle Score (HCS) in der Hypoxie-Phase zum Bestimmen des Hypoxic-Session-Index gekennzeichnet, wobei folgende Schritte durchgeführt werden:
VII. Festlegen eines HCS-Referenzwertes der Kenngröße, der kleiner als der Mittelwert der Kenngröße in der Anfangsphase ist und größer als der Hypoxie-Sicherheitswert ist;
VIII. Auftragen des HCS-Referenzwertes über die Zeit in der Hypoxiephase als HCS-Referenzlinie der Hypoxiephase;
IX. Bestimmen einer HCS-Bestimmungsfläche als Fläche zwischen der Datenkurve und der HCS-Referenzlinie in der Hypoxiephase;
X. Bestimmen einer HCS-Referenzfläche als Fläche zwischen der Referenzkurve und der HCS-Referenzlinie in der Hypoxiephase;
XI. Bestimmen einer HCS-Flächendifferenz zwischen der HCS-Referenzfläche und der HCS-Bestimmungsfläche und/oder eines HCS-Flächenverhältnis als das Verhältnis der HCS-Bestimmungsfläche und der HCS-Referenzfläche;
XII. Bestimmen des Wertes des Hypoxic-Cycle Score als Maß des HCS-Flächenverhältnis und/oder der HCS-Flächendifferenz.

Der HCS dient dem Vergleich zwischen dem Abfall der Referenzkurve und der Datenkurve in der Hypoxiephase.

Der HCS-Referenzwert liegt insbesondere zwischen 88% und 92% des Mittelwerts der Kenngröße aus der Anfangsphase, vorzugsweise bei 90% des Mittelwerts der Kenngröße aus der Anfangsphase.

Die HCS-Bestimmungsfläche wird insbesondere als Fläche zwischen der Datenkurve von dem Zeitpunkt, zu dem die Kenngröße auf der Datenkurve auf den HCS-Referenzwert abgefallen ist, bis zu dem Endzeitpunkt der Hypoxiephase und der HCS-Referenzlinie von dem Zeitpunkt, zu dem die Kenngröße auf den HCS-Referenzwert abgefallen ist, bis zu dem Endzeitpunkt der Hypoxiephase festgelegt.

Die HCS-Referenzfläche wird insbesondere als Fläche zwischen der Datenkurve von dem Zeitpunkt, zu dem die Kenngröße auf der Referenzlinie auf den HCS-Referenzwert abgefallen ist, bis zu dem Endzeitpunkt der Hypoxiephase und der HCS-Referenzlinie von dem Zeitpunkt, zu dem die Kenngröße auf den HCS-Referenzwert abgefallen ist, bis zu dem Endzeitpunkt der Hypoxiephase festgelegt.

Der Hypoxic-Cycle Score (HCS) wird insbesondere durch die absolute Abweichung der HCS-Bestimmungsfläche und der HCS-Referenzfläche bestimmt, die insbesondere durch die HCS-Flächendifferenz festgelegt ist. Alternativ wird der Hypoxic-Cycle Score durch die relative Abweichung der HCS-Bestimmungsfläche und der HCS-Referenzfläche bestimmt, bei der die HCS-Flächendifferenz auf die HCS-Referenzfläche bezogen wird. Die relative Abweichung wird insbesondere in Prozent angegeben.

Eine bevorzugte Ausgestaltung des Verfahrens ist gekennzeichnet durch das Festlegen eines Reoxygenation-Max Score (RMS) in der Reoxygenierungs-Phase zum Bestimmen des Hypoxic-Session-Index durch folgende Schritte:
XIII. Festlegen eines RMS-Sicherheitszeitpunkts nach Beginn der Reoxygenierungsphase;
XIV. Bestimmen des zu dem RMS-Sicherheitszeitpunkt zugehörigen RMS-Sicherheitswertes der Kenngröße auf der Datenkurve in der Reoxygenierungsphase;
XV. Auftragen des RMS-Sicherheitswertes über die Zeit in der Reoxygenierungsphase als RMS-Sicherheitslinie der Reoxygenierungsphase;
XVI. Bestimmen einer RMS-Bestimmungsfläche als Fläche zwischen der Datenkurve und der RMS-Sicherheitslinie in der Reoxygenierungsphase;
XVII. Bestimmen einer RMS-Referenzfläche als Fläche zwischen der Referenzkurve und der RMS-Sicherheitslinie in der Reoxygenierungsphase;
XVIII. Bestimmen einer RMS-Flächendifferenz zwischen der RMS-Referenzfläche und der RMS-Bestimmungsfläche und/oder eines RMS-Flächenverhältnis als das Verhältnis der RMS-Bestimmungsfläche und der RMS-Referenzfläche;
XIX. Bestimmen des Reoxygenation-Max Score als Maß des RMS-Flächenverhältnis und/oder der RMS-Flächendifferenz.

Der Reoxygenation-Max Score dient dem Vergleich der Referenzkurve mit der Datenkurve in der Reoxygenierungsphase.

Der RMS-Sicherheitszeitpunkts liegt vorzugweise 30 Sekunden bis 50 Sekunden, bevorzugt 45 Sekunden, nach Beginn der Reoxygenierungsphase. Der RMS-Sicherheitszeitpunkt ist bevorzugt ein Zeitpunkt, zu dem die Kenngröße zwischen 3% und 10% des Mittelwerts der Kenngröße aus der Anfangsphase annimmt.

Die RMS-Bestimmungsfläche wird insbesondere als Fläche zwischen der Datenkurve von dem RMS-Sicherheitszeitpunkt bis zu dem Endzeitpunkt der Reoxygenierungsphase und der RMS-Sicherheitslinie von dem RMS-Sicherheitszeitpunkt bis zu dem Endzeitpunkt der Reoxygenierungsphase festgelegt.

Die RMS-Referenzfläche wird insbesondere als Fläche zwischen der Referenzkurve von dem RMS-Sicherheitszeitpunkt bis zu dem Endzeitpunkt der Reoxygenierungsphase und der RMS-Sicherheitslinie von dem RMS-Sicherheitszeitpunkt bis zu dem Endzeitpunkt der Reoxygenierungsphase festgelegt.

Der Reoxygenation-Max Score (RMS) wird insbesondere durch die absolute Abweichung der RMS-Bestimmungsfläche und der RMS-Referenzfläche bestimmt, die insbesondere durch die RMS-Flächendifferenz festgelegt ist. Alternativ wird der Reoxygenation-Max Score durch die relative Abweichung der RMS-Bestimmungsfläche und der RMS-Referenzfläche bestimmt, bei der die RMS-Flächendifferenz auf die RMS-Referenzfläche bezogen wird. Die relative Abweichung wird insbesondere in Prozent angegeben.

Die Referenzkurve steigt insbesondere auf 99% des Wertes der Kenngröße an, den die Kenngröße in der Anfangsphase maximal erreichen kann.

Wenn die Kenngröße der Sauerstoffpartialdruck ist oder die Sauerstoffsättigung, so steigt die Referenzkurve insbesondere auf 99% des Sauerstoffpartialdrucks oder auf 99% der Sauerstoffsättigung als maximal gesunden Wert der Kenngröße an, bezogen auf den Fall, dass das gesamte Hämoglobin im Blut mit Sauerstoff beladen ist. Bevorzugt steigt die Referenzkurve in einem Zeitraum von 10 Sekunden bis 14 Sekunden, vorzugsweise 12 Sekunden, nach dem RMS-Sicherheitszeitpunkt auf den maximal gesunden Wert der Kenngröße an.

Eine Weiterbildung der vorgenannten Ausgestaltung des Verfahrens ist gekennzeichnet durch das Bestimmen eines User-Reoxygenation-Potentials als Maß der Differenz zwischen einem vorbestimmten Referenz-Reoxygenation-Max Score und dem in Schritt XIX bestimmten Reoxygenation-Max Score. Das User-Reoxygenation-Potential ist insbesondere die vorgenannte Differenz, ausgedrückt in Prozent. Der Referenz-Reoxygenation-Max Score beträgt insbesondere 99% des maximalen Werts, den die Kenngrö-ße in der Anfangsphase erreichen kann.

Bevorzugte Ausgestaltungen des Verfahrens sind durch das Festlegen eines Dynamic Score in der Hypoxie-Phase zum Bestimmen des Hypoxic-Session-Index gekennzeichnet, aufweisend die Schritte:
XX. Bestimmen eines DS-Referenz-Zeitpunkts, zu dem die Kenngröße in der Hypoxiephase auf einen vorgegebenen DS-Bezugswert abgefallen ist, wobei der DS-Bezugswert kleiner als der Mittelwert der Kenngröße in der Anfangsphase und größer als der Hypoxie-Sicherheitswert ist;
XXI. Bestimmen des Dynamic Score als Maß der Zeitdifferenz zwischen dem DS-Referenz-Zeitpunkt und einem vorgegebenen DS-Referenz-Zeitintervall.

Der Dynamic-Score dient zur Angabe der Reaktionszeit des Körpers eines Patienten auf einen Sauerstoffmangel, gemessen vom Anfang der Hypoxiephase, insbesondere bis eine Reaktion des Körpers an einem Sensor messbar ist.

Der vorgegebene DS-Bezugswert beträgt insbesondere 95% bis 98%, vorzugsweise 97% des Mittelwerts der Kenngröße aus der Anfangsphase. Das DS-Referenz-Zeitintervall beträgt insbesondere zwischen 40 Sekunden und 50 Sekunden, vorzugsweise 45 Sekunden. Die Zeitdifferenz zwischen dem DS-Referenz-Zeitpunkt und dem DS-Referenz-Zeitintervall wird als Wert des Dynamic-Score festgehalten. Bei negativen Werten der Zeitdifferenz und/oder Werten der Zeitdifferenz, die größer als ein vorbestimmter Wert sind, insbesondere mehr als 184 Sekunden, wird der Dynamic Score vorzugsweise auf Null gesetzt. Alternativ oder zusätzlich kann die Messzeit in der Anfangsphase und der Hypoxiephase in Zeitintervalle eingeteilt sein, wobei jedem Zeitintervall ein Hypoxie-Verbesserungspotential zugeordnet wird, sodass eine Lage des DS-Referenz-Zeitpunkts in einem bestimmten Zeitintervall einem bestimmten Hypoxie-Verbesserungspotential entspricht.

Vorteilhaft ist das Verfahren durch das Festlegen eines Reoxygenation-Impuls Score in der Reoxygenierungs-Phase zum Bestimmen des Hypoxic-Session-Index gekennzeichnet, aufweisend die Schritte:
XXII. Bestimmen eines RI-Referenz-Zeitpunkts, zu dem die Kenngröße in der Reoxygenierungsphase auf einen vorgegebenen Hyperoxie-Bezugswert angestiegen ist;
XXIII. Bestimmen des Reoxygenation-Impuls Score als Maß der Zeitdifferenz zwischen dem RI-Referenz-Zeitpunkt und einem vorgegebenen RI-Referenz-Zeitintervall.

Der Reoxygenation-Impuls Score dient zur Angabe der Reaktionszeit des Körpers eines Patienten auf ein hyperoxisches Gasgemisch, gemessen vom Anfang der Hyperoxiephase, insbesondere bis eine Reaktion des Körpers auf das hyperoxische Gasgemisch durch einen Sensor meßbar ist.

Der vorgegebene Hyperoxie-Bezugswert beträgt insbesondere 2% bis 5%, vorzugsweise 3% des Mittelwerts der Kenngröße aus der Anfangsphase. Das RI-Referenz-Zeitintervall beträgt insbesondere zwischen 40 Sekunden und 50 Sekunden, vorzugsweise 45 Sekunden.

Bei negativen Werten der Zeitdifferenz und/oder Werten der Zeitdifferenz, die größer sind als ein vorbestimmter Wert, insbesondere mehr als 184 Sekunden, wird der Reoxygenation-Impuls Score vorzugsweise auf Null gesetzt. Alternativ oder zusätzlich kann die Messzeit in der Reoxygenierungsphase in Zeitintervalle eingeteilt sein, wobei jedem Zeitintervall ein Reoxygenierungs-Verbesserungspotential zugeordnet wird, sodass eine Lage des RI-Referenz-Zeitpunkts in einem bestimmten Zeitintervall einem bestimmten Reoxygenierungs-Verbesserungspotential entspricht.

Eine Ausgestaltung des Verfahrens ist durch das Festlegen eines Oxygen-Recovery Score in der Reoxygenierungs-Phase zum Bestimmen des Hypoxic-Session-Index durch den Reoxygenierungs-Impuls Score und den Reoxygenation-Max Score, insbesondere durch Mittelung, gekennzeichnet.

Bei der Mittelung handelt es sich insbesondere um eine arithmetische Mittelung.

Vorteilhaft ist die Kenngröße des Sauerstoffgehalts der Sauerstoffpartialdruck und/oder die Sauerstoffsättigung. Diese Kenngrößen geben an, wieviel Prozent des gesamten Hämoglobins im Blut eines Patienten mit Sauerstoff beladen sind.

Eine weitere Ausgestaltung des Verfahrens ist durch die Bestimmung eines Baseline Potentials als Maß der Differenz eines vorbestimmten Idealwerts der Kenngröße der Sauerstoffsättigung und des Mittelwerts der Kenngröße der Sauerstoffsättigung aus der Anfangsphase gekennzeichnet. Der vorbestimmte Idealwert der Kenngröße der Sauerstoffsättigung beträgt insbesondere 99% des Wertes der Kenngröße, den die Kenngröße in der Anfangsphase maximal erreichen kann.

Eine vorteilhafte Ausgestaltung des Verfahrens ist durch das Festlegen einer unteren Begrenzungslinie und einer oberen Begrenzungslinie gekennzeichnet, wobei die untere Begrenzungslinie in der Hypoxiephase kleinere Werte der Kenngröße als die Referenzkurve und die obere Begrenzungslinie größere Werte der Kenngröße als die Referenzkurve zu jeweils gleichen Messzeiten aufweist, wobei die Begrenzungslinien insbesondere aus Messungen der Kenngröße an einer oder mehrerer Testpersonen bestimmt werden, wobei nur Werte der Kenngröße zur Bestimmung des Hypoxic-Session-Index berücksichtigt werden, die kleiner als der Wert der Kenngröße in der oberen Begrenzungslinie und größer als der Wert der Kenngröße in der unteren Begrenzungslinie zu dem Zeitpunkt der Messung des jeweiligen Wertes der Kenngröße sind.

Insbesondere werden zur Berechnung der in der Anmeldung genannten Referenzflächen und Bestimmungsflächen nur Datenpunkte herangezogen, die in der durch die Referenzkurven begrenzten Fläche liegen. Diese Fläche wird im Rahmen der Anmeldung als Hypoxie-Normbereich bezeichnet und legt den Bereich der gültigen Messwerte fest. Werte der Kenngröße, die sich zu dem jeweiligen Zeitpunkt außerhalb der durch die Begrenzungslinie festgelegten Fläche befinden, werden als falsche Messwerte eingestuft, zum Beispiel durch einen schlechten Kontakt zwischen dem Sensor und dem Patienten. Die Begrenzungslinien sind insbesondere vorher gemessene Datenkurven von Testpersonen mit geeigneten Werten der Kenngröße für die obere und untere Begrenzungslinie.

Eine bevorzugte Ausgestaltung des Verfahrens ist durch das Bestimmen des Hypoxic-Session-Index mit Hilfe einer Veränderung einer Pulskurve des Patienten während der Schritte III bis V gekennzeichnet. Insbesondere kann das Ansteigen oder Absinken des Pulses während des HypoxieTrainings als Indikator für die Reaktion des Körpers auf das Hypoxietraining verwendet werden.

Eine Weiterbildung der vorgenannten Ausgestaltung des Verfahrens ist gekennzeichnet durch das Bestimmen eines Heartrate-Relaxation-Score unter Auftragen einer Pulskurve über die Zeit während des Hypoxietrainings, aufweisend die folgenden Schritte:
XXIV. Bestimmen des Mittelwerts des Pulses in der Anfangsphase;
XXV. Auftragen des Mittelwerts des Pulses über die Dauer des Hypoxietrainings nach der Anfangsphase als Heartrate-Baseline parallel zu der Zeitachse, wobei die Heartrate-Baseline einen ersten Schenkel eines Relaxation-Messwinkels bildet;
XXVI. Auftragen eines zweiten Schenkels des Relaxation-Messwinkels, wobei der zweite Schenkel durch den Puls am Anfangszeitpunkt der Hypoxiephase und durch den Punkt der Pulskurve mit dem niedrigsten Wert des Pulses nach der Anfangsphase verläuft;
XXVII. Bestimmen des Heartrate-Relaxation-Score als Maß des Relaxation-Messwinkels.

Die Heartrate-Baseline wird insbesondere in der Anfangsphase bestimmt, wobei der Puls in jeder Sekunde gemessen und festgehalten wird, um aus dem Mittelwert des Pulses in der Anfangsphase die Heartrate-Baseline zu bestimmen. Der Heartrate-Relaxation-Score dient der Angabe der Veränderung des Pulses während des Hypoxie-Trainings. Dies zeigt die Entspannung des Patienten während des Hypoxie-Trainings an.

Der Maximalwert des Pulses oberhalb der Heartrate-Baseline wird zur Bestimmung eines negativen Heartrate-Relaxation-Score verwendet und der kleinste Wert des Pulses unterhalb der Heartrate-Baseline zur Bestimmung eines positiven Heartrate-Relaxation-Score verwendet. Insbesondere der positive Heartrate-Relaxation-Score wird zur Bestimmung der Reaktion des Körpers des Patienten auf das HypoxieTraining verwendet.

Eine Ausgestaltung des Verfahrens ist gekennzeichnet durch eine oder mehrere Wiederholungen der Schritte III bis V vor der Bestimmung des Hypoxic-Session-Index gemäß Schritt VI. Bei jeder Wiederholung, insbesondere als Zyklus bezeichnet, lassen sich einer oder mehrerer der vorgenannten Indices bestimmen, um daraus Mittelwerte für den jeweiligen Index zu bilden oder Werte der jeweiligen Indices zu vergleichen, um in einem Zyklus falsch gemessene Werte zu erkennen.

Eine Vorrichtung zur Durchführung eines Verfahrens nach einer der vorgenannten Ausgestaltungen weist eine Maske zur Zuführung des hypoxischen, normoxischen und/oder hyperoxischen Gasgemisches an den Patienten, eine Steuerung zum Steuern der Vorrichtung und einen Fingerclip zur Messung des Pulses und/oder der Kenngröße auf.

Durch eine solche Vorrichtung lassen sich zur Bestimmung der Reaktion des Körpers eines Patienten geeignete Indices quantitativ erfassen.

Eine Ausführungsform der Vorrichtung weist eine mobile Anwendung zur Darstellung des Hypoxic-Session-Index und/oder mindestens einem der Indices zur Bestimmung des Hypoxic-Session-Index gemäß einer der vorgenannten Ausgestaltungen auf. Der Hypoxic-Session-Index kann durch Farben einer Farbskala dargestellt werden, um so eine rasche Erfassung der Reaktion des Körpers eines Patienten auf das Hypoxietraining zu ermöglichen. Alternative Ausführungsformen der Vorrichtung weisen eine stationäre Anwendung zur Darstellung des Hypoxic-Session-Index und/oder mindestens einem der Indices zur Bestimmung des Hypoxic-Session-Index gemäß einer der vorgenannten Ausgestaltungen auf.

Weitere Vorteile der Erfindung ergeben sich aus der Beschreibung und der Zeichnung. Ebenso können die vorstehend genannten und die noch weiter ausgeführten Merkmale jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt schematisch eine Vorrichtung zur Messung der Reaktion eines Patienten auf ein Hypoxietraining;
- Fig. 2: schematisch eine Übersicht von durch die Vorrichtung gemessenen Einträgen, durch die ein Angabewert, umfassend einen Hypoxic-Session-Index und/oder einen Heartrate-Relaxation Score als Maß der Reaktion eines Patienten auf ein Hypoxietraining, bestimmt wird;
- Fig. 3: zeigt schematisch ein Verfahren zur Messung des Hypoxic-Session-Index HSI;
- Fig. 4: zeigt schematisch eine Datenkurve einer Kenngröße, die in dem Verfahren aufgenommen wird;
- Fig. 5: zeigt schematisch die Datenkurve bis zu einem DS-Referenz-Zeitpunkt, bei dem die Kenngröße auf einen vorgegebenen DS-Bezugswert abgefallen ist;
- Fig. 6: zeigt schematisch eine untere Begrenzungslinie und eine obere Begrenzungslinie der Kenngröße in einer Hypoxiephase;
- Fig. 7: zeigt schematisch die Datenkurve vom Beginn einer Reoxygenierungsphase bis zu einem RI-Referenz-Zeitpunkt, bei dem die Kenngröße auf einen Hyperoxie-Bezugswert angestiegen ist;
- Fig. 8: zeigt schematisch die Referenzkurve sowie eine erste Datenkurve und eine zweite Datenkurve in der Reoxygenierungsphase oberhalb eines RMS-Sicherheitswerts;
- Fig. 9: zeigt schematisch einen Puls des Patienten, der in Form einer Pulskurve über die Messzeit aufgetragen ist;
- Fig. 10: zeigt schematisch die Vorrichtung mit einer Anzeige.

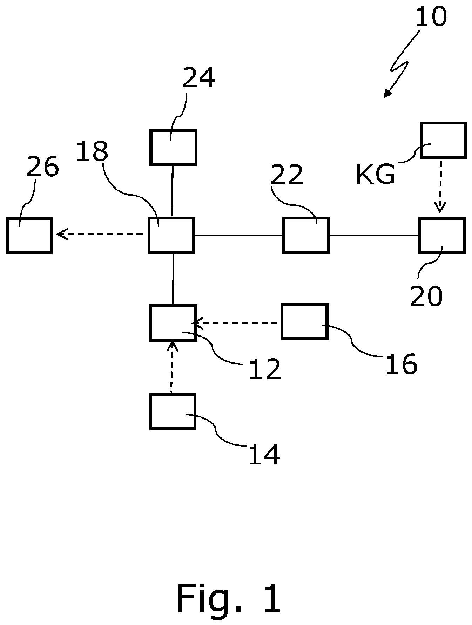

**Fig. 1** zeigt schematisch eine Vorrichtung **10** zur Messung der Reaktion eines Patienten auf ein Hypoxietraining. Die Vorrichtung weist eine Maske **12** zur Zuführung eines hypoxischen Gasgemisches **14,** eines normoxischen und/oder hyperoxischen Gasgemisches **16** an einen Patienten (nicht gezeigt) auf.

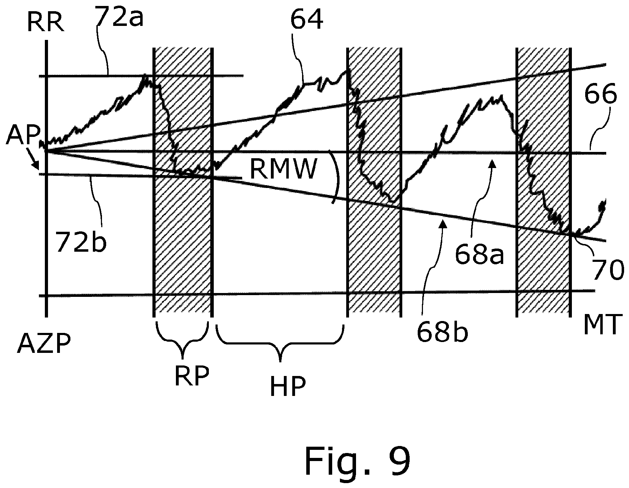

Außerdem verfügt die Vorrichtung über eine Steuerung **18** zum Steuern der Vorrichtung 10 und einen Fingerclip **20** sowie einen, insbesondere in dem Fingerclip 20 angeordneten, Sensor **22** zur Messung eines Pulses **RR** (siehe Fig. 9) des Patienten und/oder einer Kenngröße **KG,** die die Sauerstoffsättigung des Bluts des Patienten kennzeichnet, zum Beispiel des Sauerstoffpartialdrucks. Die Vorrichtung 10 umfasst eine mobile Anwendung **24** zur Darstellung eines Angabewerts **26,** durch den die Reaktion des Körpers des Patienten auf das Hypoxietraining quantitativ erfasst wird.

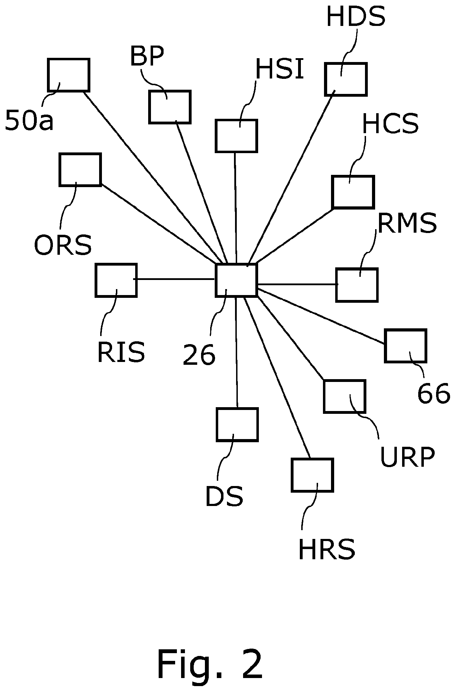

**Fig. 2** zeigt schematisch eine Übersicht der Einträge, durch die der Angabewert 26 bestimmt wird. Diese Einträge umfassen einen Hypoxic-Session-Index **HSI,** einen Hypoxic-Cycle Score **HCS,** einen Reoxygenation-Max Score **RMS,** ein User-Reoxygenation-Potential **URP,** einen Dynamic Score **DS,** einen Reoxygenation-Impuls Score **RIS,** einen Oxygen-Recovery Score **ORS,** ein Baseline Potential **BP,** eine Hypoxie-Baseline **50a,** eine Heartrate-Baseline **66,** einen Heartrate-Relaxation-Score **HRS** und/oder einen Heartrate-Dynamic-Score **HDS.** Diese Einträge oder Indices können zusammengefasst, zum Beispiel in Form einer Mittelung, oder getrennt, zum Beispiel in Listenform, angezeigt werden. Insbesondere ergibt sich der Hypoxic-Session-Index HSI aus einem oder mehreren der übrigen vorgenannten Indices, zum Beispiel durch Mittelung dieser Indices.

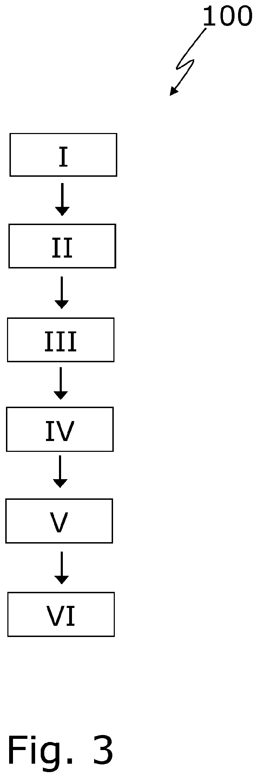

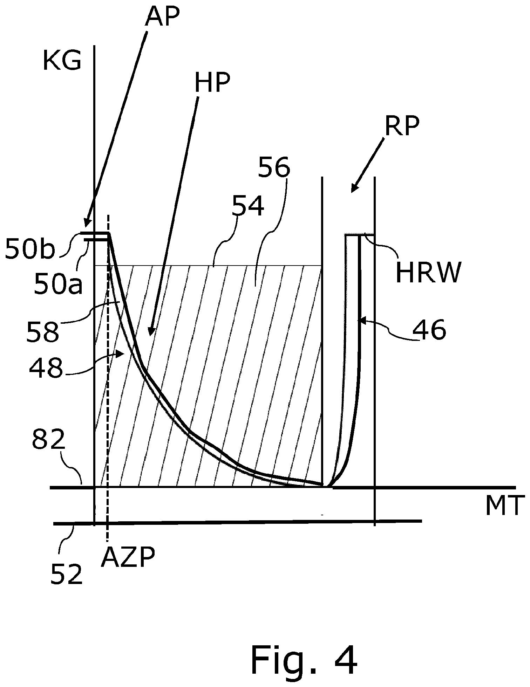

**Fig. 3** zeigt schematisch ein Verfahren **100** zur Messung der Reaktion eines Patienten auf ein Hypoxietraining. In einem ersten Schritt I erfolgt ein Versorgen des Patienten, insbesondere in einer Ruheposition, mit dem normoxischen Gasgemisch 16 in einer Anfangsphase **AP** (siehe Fig. 4) über einen vorgegebenen ersten Zeitraum, insbesondere über einen ersten Zeitraum von 20 Sekunden bis 40 Sekunden, vorzugsweise 30 Sekunden. In einem zweiten Schritt II wird der Mittelwert der Kenngröße KG in der Anfangsphase AP berechnet. Nach der Anfangsphase AP wird der Patient in einem dritten Schritt III über einen vorgegebenen Hypoxie-Zeitraum mit einem hypoxischen Gasgemisch 14 in einer Hypoxiephase **HP** (siehe Fig. 4) versorgt, bis die Kenngröße KG auf einen Hypoxie-Wert **82** abfällt, der größer als ein vorgegebener Hypoxie-Sicherheitswert **52** ist (siehe Fig. 4). In einem vierten Schritt IV wird der Patient (nicht gezeigt) über einen vorgegebenen Hyperoxie-Zeitraum mit einem normoxischen oder hyperoxischen Gasgemisch 16 in einer Reoxygenierungsphase **RP** (siehe Fig. 4) versorgt, wobei die Kenngröße KG einen Hyperoxie-Referenzwert **HRW** (siehe Fig. 4) erreicht, und in einem sich daran anschließenden, vorbestimmten Abschlusszeitraum. In einem fünften Schritt V wird die Kenngröße KG über die Zeit in der Anfangsphase AP, der Hypoxiephase HP und der Reoxygenierungsphase RP in Form einer Datenkurve **46** (siehe Fig. 4) aufgetragen. In einem sechsten Schritt VI wird der Hypoxic-Session-Index HSI aus einer Abweichung der Datenkurve 46 von einer vorbestimmten Referenzkurve **48** (siehe Fig. 4) bestimmt.

**Fig. 4** zeigt schematisch eine Datenkurve 46 der Kenngröße KG, die in dem Verfahren 100 zur Messung des Hypoxic-Session-Index 26 über eine Messzeit MT in der Anfangsphase AP, der Hypoxiephase HP und der Reoxygenierungsphase RP aufgenommen wird. Dargestellt ist auch eine vorbestimmte Referenzkurve 48, mit der die Datenkurve 46 verglichen wird. Hypoxie-Baselines **50a** der Referenzkurve 48 und **50b** der Datenkurve 46 ergeben sich durch Auftragen des Mittelwerts von Messwerten der Kenngröße KG über die Messzeit MT in der Anfangsphase AP des Verfahrens 100. In der Reoxygenierungsphase RP steigen die Referenzkurve 48 und die Datenkurve 46 auf den Hyperoxie-Referenzwert HRW an, der hier schematisch als der gleiche Wert für die Referenzkurve 48 und die Datenkurve 46 dargestellt ist. Die Hpoxiephase HP beginnt am Anfangszeitpunkt **AZP.** In der Hypoxiephase wird der Patient über einen vorgegebenen Hypoxie-Zeitraum mit einem hypoxischen Gasgemisch 14 versorgt, bis die Kenngröße KG auf den Hypoxie-Wert 82 abfällt, der größer ist als der vorgegebene Hypoxie-Sicherheitswert 52, der als Hypoxie-Sicherheitswert-Linie **52** über die Messzeit MT aufgetragen ist.

Ein vorbestimmter HCS-Referenzwert der Kenngröße KG ist kleiner als der Mittelwert der Kenngröße in der Anfangsphase und größer als der Hypoxie-Sicherheitswert 52. Der HCS-Referenzwert ist als HCS-Referenzlinie **54** der Hypoxiephase über die Messzeit MT dargestellt. Eine HCS-Bestimmungsfläche **56** ist als Fläche zwischen der Datenkurve 46 und der HCS-Referenzlinie 54 in der Hypoxiephase HP festgelegt. Eine HCS-Referenzfläche **58** ist als Fläche zwischen der Referenzkurve 48 und der HCS-Referenzlinie 54 in der Hypoxiephase bestimmt, deren Ende durch eine senkrechte Linie dargestellt ist. Der Wert des Hypoxic-Cycle Score HCS ist als Maß des Verhältnisses und/oder der Differenz der HCS-Bestimmungsfläche 56 und der HCS-Referenzfläche 58 definiert.

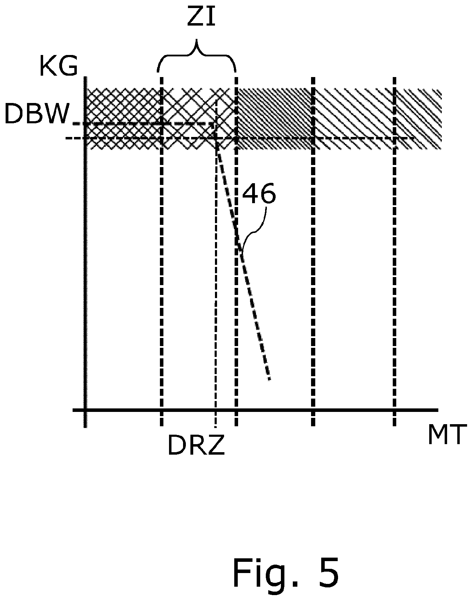

**Fig. 5** zeigt schematisch die Datenkurve 46 in der Anfangsphase AP und in der Hypoxiephase HP (sieh Fig. 4) bis zu einem DS-Referenz-Zeitpunkt **DRZ** der Messzeit MT, bei dem die Kenngröße KG auf einen vorgegebenen DS-Bezugswert **DBW** abgefallen ist. Schematisiert ist die Datenkurve 46 in der Anfangsphase AP und bis zu dem Zeitpunkt, zu dem sie abzufallen beginnt, als gerade Linie dargestellt.

Der DS-Bezugswert DBW ist kleiner als der Mittelwert der Kenngröße KG in der Anfangsphase AP und größer als der Hypoxie-Sicherheitswert 52 (siehe Fig. 4). Der DS-Bezugswert DBW beträgt insbesondere 96% bis 98% des Mittelwertes der Kenngröße KG aus der Anfangsphase AP. Die Zeitdifferenz zwischen dem DS-Referenz-Zeitpunkt DRZ und einem vorgegebenen DS-Referenz-Zeitintervall, insbesondere einem DS-Referenz-Zeitintervall von 40 Sekunden bis 50 Sekunden, wird als Wert des Dynamic-Score DS festgehalten, gegebenenfalls geteilt durch einen Zahlenfaktor, zum Beispiel 100. Bei negativen Werten der Zeitdifferenz und/oder Werten der Zeitdifferenz, die größer als ein vorbestimmter Wert sind, insbesondere mehr als 184 Sekunden, wird der Dynamic Score DS vorzugsweise auf Null gesetzt.

Alternativ oder zusätzlich kann die Messzeit in der Anfangsphase AP und der Hypoxiephase HP in Zeitintervalle **ZI** eingeteilt sein, wobei jedem Zeitintervall ZI ein Hypoxie-Verbesserungspotential, dargestellt durch unterschiedliche Schraffierungen in den Zeitintervallen ZI, zugeordnet wird, sodass eine Lage des DS-Referenz-Zeitpunkts DRZ in einem bestimmten Zeitintervall einem bestimmten Hypoxie-Verbesserungspotential entspricht.

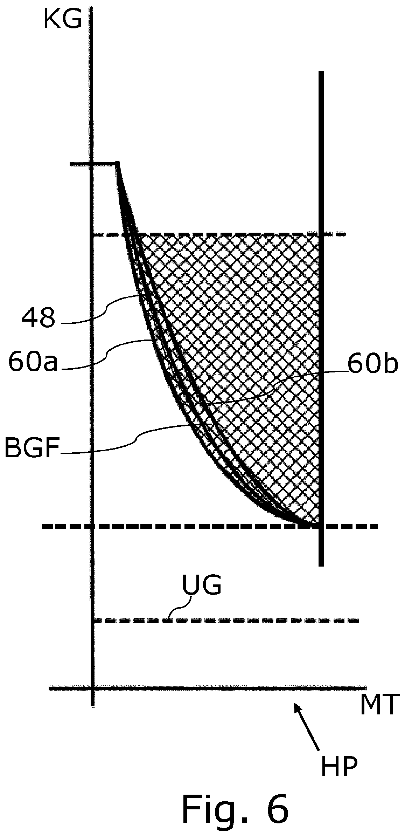

**Fig. 6** zeigt schematisch eine untere Begrenzungslinie **60a** und eine obere Begrenzungslinie **60b** der Kenngröße KG in der Hypoxiephase HP. Die untere Begrenzungslinie 60a in der Hypoxiephase HP weist zu der jeweiligen Messzeit MT kleinere Werte der Kenngröße KG als die Referenzkurve 48 und die obere Begrenzungslinie 60b größere Werte der Kenngröße KG als die Referenzkurve 48 auf. Nur Werte der Kenngröße KG, die in der durch die Begrenzungslinie begrenzten Fläche **BGF** liegen, werden insbesondere miteinander verbunden und zur Bestimmung der in der Anmeldung genannten Flächen und Indices herangezogen. Die mit **UG** gekennzeichnete gestrichelte Linie stellt eine untere Grenze der Kenngröße KG in der Hypoxiephase HP dar, die als Hypoxie-Sicherheitswert 52 (siehe Fig. 4) gewählt werden kann.

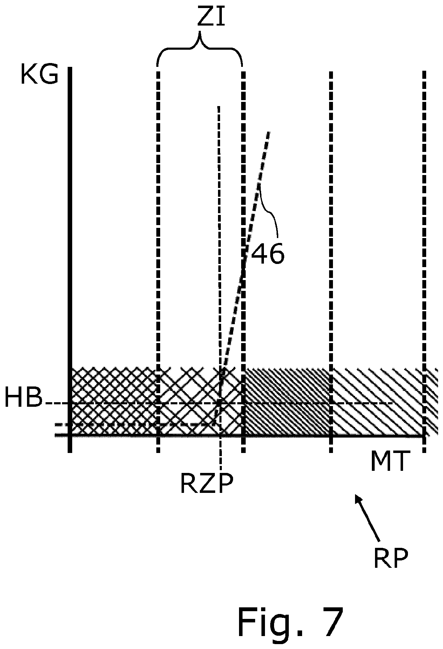

**Fig. 7** zeigt schematisch die Datenkurve 46 vom Beginn der Reoxygenierungsphase RP bis zu einem RI-Referenz-Zeitpunkt **RZP** der Messzeit MT, bei dem die Kenngröße KG auf einen vorgegebenen Hyperoxie-Bezugswert **HB** angestiegen ist. Schematisiert wird die Datenkurve 46 vom Beginn der Reoxygenierungsphase RP bis zu dem Zeitpunkt, bei dem die Kenngröße KG ansteigt, als gerade Linie dargestellt. Der Hyperoxie-Bezugswert HB beträgt insbesondere 2% bis 4% des Mittelwerts der Kenngröße KG aus der Anfangsphase AP.

Die Zeitdifferenz zwischen dem RI-Referenz-Zeitpunkt RZP und einem vorgegebenen RI-Referenz-Zeitintervall, insbesondere ein RI-Referenz-Zeitintervall von 40 Sekunden bis 50 Sekunden, wird als Wert des Reoxygenation-Impuls Score RIS festgehalten. Bei negativen Werten der Zeitdifferenz und/oder Werten der Zeitdifferenz, die größer als ein vorbestimmter Wert sind, insbesondere mehr als 184 Sekunden, wird der Reoxygenation-Impuls Score RIS vorzugsweise auf Null gesetzt. Alternativ oder zusätzlich kann die Messzeit MT in der Reoxygenierungsphase RP in Zeitintervalle ZI eingeteilt sein, wobei jedem Zeitintervall ZI ein Reoxygenierungs-Verbesserungspotential, dargestellt durch unterschiedliche Schraffierungen, zugeordnet wird, sodass eine Lage des RI-Referenz-Zeitpunkts RZP in einem bestimmten Zeitintervall ZI einem bestimmten Reoxygenierungs-Verbesserungspotential entspricht.

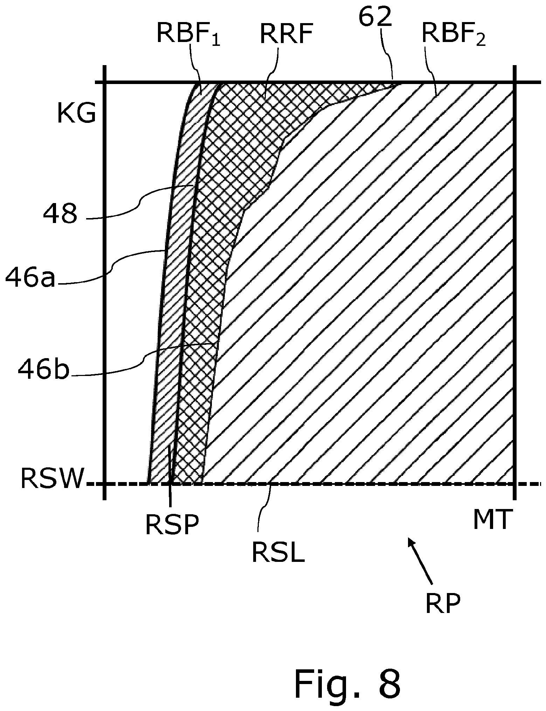

**Fig. 8** zeigt schematisch eine Referenzkurve 48 sowie eine erste Datenkurve **46a** und eine zweite Datenkurve **46b** in der Reoxygenierungsphase RP oberhalb eines RMS-Sicherheitswerts **RSW,** dargestellt über die Messzeit MT als gestrichelte, waagerechte RMS-Sicherheitslinie **RSL.** Dabei ist der RMS-Sicherheitswert ein Wert der Kenngröße KG auf der Referenzkurve 48 zu einem RMS-Sicherheitszeitpunkt **RSP** nach Beginn der Reoxygenierungsphase RP.

Hierbei erreicht die erste Datenkurve 46a den RMS-Sicherheitswert RSW zu einem früheren Zeitpunkt als die Referenzkurve 48 und die zweite Datenkurve 46b erreicht den RMS-Sicherheitswert RSW zu einem späteren Zeitpunkt als die Referenzkurve 48. Die waagerechte Linie 62 kennzeichnet schematisch einen maximalen Wert der Kenngröße KG, den die Referenzkurve 48, die erste Datenkurve 46a und die zweite Datenkurve 46b in der Reoxygenierungsphase RP in diesem Ausführungsbeispiel annnehmen. Die gekreuzt schraffierte Fläche und die grob schraffierte Fläche unterhalb der Referenzkurve bilden zusammen die RMS-Referenzfläche **RRF.** Der Reoxygenation-Max Score RMS der ersten Datenkurve 46a wird als das Verhältnis der Gesamtfläche von fein schraffierter Fläche, gekreuzt schraffierter Fläche und grob schraffierter Fläche unterhalb der ersten Datenkurve als RMS-Bestimmungsfläche **RBF₁** und der RMS-Referenzfläche RRF bestimmt. Der Reoxygenation-Max Score RMS der zweiten Datenkurve wird als das Verhältnis der der grob schraffierten Fläche unterhalb der zweiten Datenkurve als RMS-Bestimmungsfläche **RBF₂** und der RMS-Referenzfläche RRF bestimmt.

Das jeweilige User-Reoxygenation-Potential URP (siehe Fig. 2) der ersten und zweiten Datenkurve ist als Maß der Differenz zwischen einem vorbestimmten Referenz-Reoxygenation-Max Score, insbesondere einem Wert zwischen 0,97 und 1, vorzugsweise 0,99, und dem jeweiligen derart bestimmten Reoxygenation-Max Score RMS der ersten und zweiten Datenkurve, festgelegt. Das jeweilige URP stellt ein Maß der Abweichung der ersten bzw. zweiten Datenkurve 46a, 46b von der Referenzkurve 48 in der Reoxigierungsphase RP dar.

**Fig. 9** zeigt schematisch den Puls RR eines Patienten (nicht gezeigt) über die Messzeit MT in Form einer Pulskurve **64.** Der Mittelwert des Pulses RR in der Anfangsphase AP ist über die Dauer des Hypoxietrainings nach der Anfangsphase als Heartrate-Baseline **66** parallel zu der Zeitachse der Messzeit MT aufgetragen. Dabei bildet die Heartrate-Baseline 66 einen ersten Schenkel **68a** eines Relaxation-Messwinkels **RMW.** Ein zweiter Schenkel **68b** des Relaxation-Messwinkels RMW verläuft durch den Wert des Pulses RR am Anfangszeitpunkt **AZP** einer ersten Hypoxiephase HP und durch den Punkt **70** der Pulskurve 64 mit dem niedrigsten Wert des Pulses RR nach der Anfangsphase AP. Der Heartrate-Relaxation-Score HRS ist als Maß des Relaxation-Messwinkels RMW festgelegt. Insbesondere sind die Schritte II bis V des Verfahrens vor der Bestimmung des Heartrate-Relaxation-Score mehrfach wiederholt worden, wobei die Hypoxiephasen HP, dargestellt durch nicht schraffierte Flächen, und die Reoxygenierungsphasen RP, die durch schraffierte Flächen dargestellt sind, einander abwechseln. Pro Zyklus, der eine Hypoxiephase und eine nacholgende Reoxygenierungsphase umfasst, wird ein Heartrate-Dynamic Score HDS als Differenz des maximalen Werte des Pulses RR in dem Zyklus, angezeigt durch eine waagerechte Linie **72a,** und des minimalen Werte des Pulses RR in dem Zyklus, angezeigt durch eine waagerechte Linie **72b,** bestimmt.

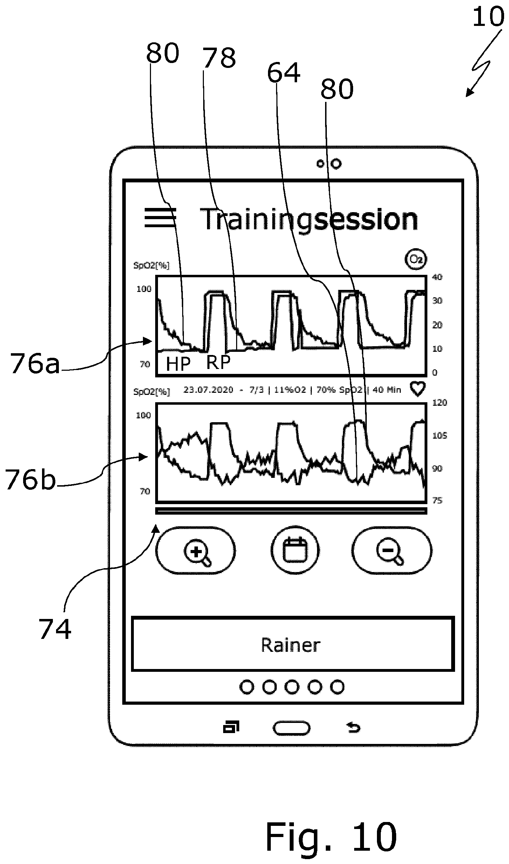

**Fig. 10** zeigt schematisch die Vorrichtung 10 mit einer Anzeige **74.** Die Anzeige 74 weist ein erstes Anzeigefeld **76a** auf, in dem der Sauerstoff O₂, der einem Patienten über die Messzeit MT zugeführt wird, als Sauerstoffkurve **78** dargestellt ist. Gezeigt ist insbesondere die wechselnde Sauerstoffmenge in der Hypoxiephase HP und in der Reoxygenierungsphase RP. Auf eine Hypoxiephase HP folgt jeweils eine Reoxygenierungsphase RP. Dargestellt sind auch die zugehörigen Werte der Kenngröße, die die Sauerstoffsättigung des Bluts eines Patienten kennzeichnet, in Form einer Sauerstoffpartialdruckkurve **80.** Die linke Skala des ersten Anzeigefelds 76a bezieht sich auf den Sauerstoffpartialdruck SpO2, angegeben in Prozent. Die rechte Skala bezieht sich auf die Sauerstoffmenge O₂ in dem zugeführten Gasgemisch, angegeben in vol%. Die Sauerstoffmenge O₂ in dem zugeführten Gasgemisch schwankt insbesondere zwischen 7,5 vol% bis 17 vol% in der Hypoxiephase HP und 20 vol% bis 35 vol%, insbesondere 20,9 vol% bis 32 vol%, vorzugsweise 25 vol% bis 30 vol%, in der Reoxygenierungsphase RP. Die waagerechte Achse gibt die Messzeit MT während des Hypoxietrainings an.

Die Anzeige 74 weist ein zweites Anzeigefeld 76b auf, in dem die Werte des Sauerstoffpartialdrucks SpO2 als die Sauerstoffpartialdruckkurve 80 und des Pulses RR des Patienten als Pulskurve 64 über die Messzeit MT aufgetragen sind. Die linke Skala des zweiten Anzeigefelds 76b bezieht sich den Sauerstoffpartialdruck SpO2, angegeben in Prozent. Die rechte Skala bezieht sich auf den Puls RR, angegeben in bpm (beats per minute). Die waagerechte Achse gibt die Messzeit MT während des Hypoxietrainings an.

Unter Vornahme einer Zusammenschau aller Figuren der Zeichnung betrifft die Erfindung ein Verfahren 100 zur Messung eines Hypoxic-Session-Index HSI als Maß der Reaktion eines Patienten auf ein Hypoxietraining, unter Messung einer den Sauerstoffgehalt im Blut des Patienten kennzeichnenden Kenngröße KG. Die Kenngröße KG ist insbesondere die Sauerstoffsättigung und/oder der Sauerstoffpartialdruck SpO2. Der Patient wird zunächst in einer Anfangsphase AP mit einem normoxischen Gasgemisch 16 versorgt. Anschließend wird dem Patienten in einer Hypoxiephase HP ein hypoxisches Gasgemisch 14 über einen vorgegebenen Hypoxie-Zeitraum zugeführt. Anschließend erhält der Patient in einer Reoxygenierungsphase RP, insbesondere über einen Zeitraum von 1 Minute bis 10 Minuten, ein normoxisches oder hyperoxisches Gasgemisch 16. Die Reoxygenierungsphase RP erstreckt sich von dem Zeitpunkt, ab dem dem Patienten das normoxische oder hyperoxische Gasgemisch 16 zugeführt wird, über einen vorgegebenen Hyperoxie-Zeitraum , wobei die Kenngröße KG einen Hyperoxie-Referenzwert HRW der Kenngröße KG erreicht, und über einen sich daran anschließenden vorbestimmten Abschlusszeitraum. Aus den Unterschieden zwischen einer Datenkurve 46, 46a, 46b, aufweisend die über die jeweilige Messzeit MT aufgetragenen Messwerte der Kenngrö-ße KG, und einer vorbestimmten Referenzkurve 48 wird der Hypoxic-Session-Index HSI ermittelt.

### Bezugszeichenliste

- 10: Vorrichtung
- 12: Maske
- 14: hypoxisches Gasgemisch
- 16: normoxisches und/oder hyperoxisches Gasgemisch
- 18: Steuerung
- 20: Fingerclip
- 22: Sensor
- 24: Mobile Anwendung
- 26: Angabewert

- 45: Heartrate-Dynamic Score
- 46, 46a,b: Datenkurve
- 48: Referenzkurve
- 50a,b: Hypoxie-Baselines
- 52: Hypoxie-Sicherheitswert-Linie
- 54: HCS-Referenzlinie
- 56: HCS-Bestimmungsfläche
- 58: HCS-Referenzfläche
- 60a,b: untere, obere Begrenzungslinie
- 62: maximaler Wert der Referenzkurve und ersten, zweiten Datenkurve in der Reoxiginierungsphase
- 64: Pulskurve
- 66: Heartrate-Baseline
- 68a,b: Schenkel
- 70: Punkt der Pulskurve 64 mit dem niedrigsten Wert des Pulses
- 72a,b: maximaler, minimaler Wert des Pulses
- 74: Anzeige
- 76a,b: Anzeigefelder
- 78: Sauerstoff kurve
- 80: Sauerstoffpartialdruckkurve

- 82: Hypoxie-Wert

- 100: Verfahren

- AP: Anfangsphase
- AZP: Anfangszeitpunkt der ersten Hypoxiephase
- BGF: durch die Begrenzungslinie begrenzten Fläche
- BP: Baseline Potential
- DBW: DS-Bezugswert
- DRZ: DS-Referenz-Zeitpunkt
- DS: Dynamic Score
- HB: Hyperoxie-Bezugswert
- HCS: Hypoxic-Cycle Score
- HDS: Heartrate-Dynamic Score
- HP: Hypoxiephase
- HRS: Heartrate-Relaxation-Score
- HRW: Hyperoxie-Referenzwert
- HSI: Hypoxic-Session-Index
- KG: Kenngröße
- MT: Messzeit
- ORS: Oxygen-Recovery Score
- RBF: RMS-Bestimmungsfläche
- RIS: Reoxygenation-Impuls Score
- RMS: Reoxygenation-Max Score
- RMW: Relaxation-Messwinkel
- RP: Reoxiginierungsphase
- RR: Puls
- RRF: RMS-Referenzfläche
- RSL: RMS-Sicherheitslinie
- RSP: RMS-Sicherheitszeitpunkt
- RSW: RMS-Sicherheitswert
- RZP: RI-Referenz-Zeitpunkt
- SBL: Hypoxie-Baseline (SPO2-Baseline)
- UG: Untere Grenze der Kenngröße in der Hypoxiephase
- URP: User-Reoxygenation-Potential
- ZI: Zeitintervalle

## Patentansprüche

1. Verfahren (100) zur Messung eines Hypoxic-Session-Index (HSI) als Maß der Reaktion eines Patienten auf ein Hypoxietraining, unter Messung einer Kenngröße (KG), die die Sauerstoffsättigung des Bluts des Patienten kennzeichnet, aufweisend die Schritte:
I. Versorgen des Patienten mit einem normoxischen Gasgemisch (16) in einer Anfangsphase (AP) über einen vorgegebenen ersten Zeitraum;
II. Bestimmen des Mittelwerts der Kenngröße (KG) in der Anfangsphase;
III. Versorgen des Patienten mit einem hypoxischen Gasgemisch (14) in einer Hypoxiephase (HP) über einen vorgegebenen Hypoxie-Zeitraum;
IV. Versorgen des Patienten mit dem normoxischen oder hyperoxischen Gasgemisch (16) in einer Reoxygenierungsphase (RP) über einen vorgegebenen Hyperoxie-Zeitraum und in einem sich daran anschließenden, vorbestimmten Abschlusszeitraum;
V. Auftragen der Kenngröße (KG) über die Zeit in der Anfangsphase (AP), der Hypoxiephase (HP) und der Reoxygenierungsphase (RP) in Form einer Datenkurve (46, 46a, 46b);
VI. Bestimmen des Hypoxic-Session-Index aus einer Abweichung der Datenkurve (46, 46a, 46b) von einer vorbestimmten Referenzkurve (48),
wobei die Referenzkurve (48) eine Datenkurve (46, 46a, 46b) ist, die nach den Schritten I bis V bei einer Referenzperson bestimmt wird, oder eine Datenkurve (46, 46a, 46b) ist, die durch eine Mittelung von Datenkurven (46, 46a, 46b) mehrerer Referenzpersonen berechnet wird, wobei die Datenkurven (46, 46a, 46b) nach den Schritten I bis V bei den Referenzpersonen bestimmt wird.

2. Verfahren nach Anspruch 1, **gekennzeichnet durch** das Festlegen eines Hypoxic-Cycle Score (HCS) in der Hypoxie-Phase (HP) zum Bestimmen des Hypoxic-Session-Index (HSI) durch folgende Schritte:
VII. Festlegen eines HCS-Referenzwertes (54) der Kenngröße (KG), der kleiner als der Mittelwert der Kenngröße (KG) in der Anfangsphase (AP) ist und größer als ein Hypoxie-Sicherheitswert (52) ist;
VIII. Auftragen des HCS-Referenzwertes (54) über die Zeit in der Hypoxiephase (HP) als HCS-Referenzlinie (54) der Hypoxiephase (HP);
IX. Bestimmen einer HCS-Bestimmungsfläche (56) als Fläche zwischen der Datenkurve (46, 46a, 46b) und der HCS-Referenzlinie (54) in der Hypoxiephase (HP);
X. Bestimmen einer HCS-Referenzfläche (58) als Fläche zwischen der Referenzkurve (48) und der HCS-Referenzlinie (54) in der Hypoxiephase (HP);
XI. Bestimmen einer HCS-Flächendifferenz zwischen der HCS-Referenzfläche (58) und der HCS-Bestimmungsfläche (56) und/oder eines HCS-Flächenverhältnis als das Verhältnis der HCS-Bestimmungsfläche (56) und der HCS-Referenzfläche (58);
XII. Bestimmen des Wertes des Hypoxic-Cycle Score (HCS) als Maß des HCS-Flächenverhältnis und/oder der HCS-Flächendifferenz.

3. Verfahren nach einem der Ansprüche 1 bis 2, **gekennzeichnet durch** das Festlegen eines Reoxygenation-Max Score (RMS) in der Reoxygenierungs-Phase (RP) zum Bestimmen des Hypoxic-Session-Index (HSI) durch folgende Schritte:
XIII. Festlegen eines RMS-Sicherheitszeitpunkts (RSP) nach Beginn der Reoxygenierungsphase (RP);
XIV. Bestimmen des zu dem RMS-Sicherheitszeitpunkt (RSP) zugehörigen RMS-Sicherheitswertes (RSW) der Kenngröße (KG) auf der Datenkurve (46, 46a, 46b) in der Reoxygenierungsphase (RP);
XV. Auftragen des RMS-Sicherheitswertes (RSW) über die Zeit (MT) in der Reoxygenierungsphase (RP) als RMS-Sicherheitslinie (RSL) der Reoxygenierungsphase (RP);
XVI. Bestimmen einer RMS-Bestimmungsfläche (RBF₁, RBF₂) als Fläche zwischen der Datenkurve (46, 46a, 46b) und der RMS-Sicherheitslinie (RSL) in der Reoxygenierungsphase (RP);
XVII. Bestimmen einer RMS-Referenzfläche (RRF) als Fläche zwischen der Referenzkurve (48) und der RMS-Sicherheitslinie (RSL) in der Reoxygenierungsphase (RP);
XVIII. Bestimmen einer RMS-Flächendifferenz zwischen der RMS-Referenzfläche (RRF) und der RMS-Bestimmungsfläche (RBF₁, RBF₂) und/oder eines RMS-Flächenverhältnis als das Verhältnis der RMS-Bestimmungsfläche (RBF₁, RBF₂) und der RMS-Referenzfläche (RRF);
XIX. Bestimmen des Reoxygenation-Max Score (RMS) als Maß des RMS-Flächenverhältnis und/oder der RMS-Flächendifferenz.

4. Verfahren nach Anspruch 3, **gekennzeichnet durch** das Bestimmen eines User-Reoxygenation-Potentials (URP) als Maß der Differenz zwischen einem vorbestimmten Referenz-Reoxygenation-Max Score (RMS) und dem in Schritt XIX bestimmten Reoxygenation-Max Score (RMS).

5. Verfahren nach einem der vorhergehenden Ansprüche, **gekennzeichnet durch** das Festlegen eines Dynamic Score (DS) in der HypoxiePhase (HP) zum Bestimmen des Hypoxic-Session-Index (HSI), aufweisend die Schritte:
XX. Bestimmen eines DS-Referenz-Zeitpunkts (DRZ), zu dem die Kenngröße (KG) in der Hypoxiephase (HP) auf einen vorgegebenen DS-Bezugswert (DBW) abgefallen ist, wobei der DS-Bezugswert (DBW) kleiner als der Mittelwert der Kenngröße (KG) in der Anfangsphase (AP) und größer als der Hypoxie-Sicherheitswert (52) ist;
XXI. Bestimmen des Dynamic Score (DS) als Maß der Zeitdifferenz zwischen dem DS-Referenz-Zeitpunkt (DRZ) und einem vorgegebenen DS-Referenz-Zeitintervall.

6. Verfahren nach einem der vorhergehenden Ansprüche, **gekennzeichnet durch** das Festlegen eines Reoxygenation-Impuls Score (RIS) in der Reoxygenierungs-Phase (RP) zum Bestimmen des Hypoxic-Session-Index (HSI), aufweisend die Schritte:
XXII. Bestimmen eines RI-Referenz-Zeitpunkts (RZP), zu dem die Kenngröße (KG) in der Reoxygenierungsphase (RP) auf einen vorgegebenen Hyperoxie-Bezugswert (HB) angestiegen ist;
XXIII. Bestimmen des Reoxygenation-Impuls Score (RIS) als Maß der Zeitdifferenz zwischen dem RI-Referenz-Zeitpunkt (RZP) und einem vorgegebenen RI-Referenz-Zeitintervall.

7. Verfahren nach einem der vorhergehenden Ansprüche, **gekennzeichnet durch** das Festlegen eines Oxygen-Recovery Score (ORS) in der Reoxygenierungs-Phase (RP) zum Bestimmen des Hypoxic-Session-Index (HSI) durch den Reoxygenierungs-Impuls Score (RIS) und den Reoxygenation-Max Score (RMS), insbesondere durch Mittelung.

8. Verfahren nach einem der vorhergehenden Ansprüche, **gekennzeichnet durch** die Bestimmung eines Baseline Potentials (BP) als Maß der Differenz eines vorbestimmten Idealwerts der Kenngröße (KG) der Sauerstoffsättigung und des Mittelwerts der Kenngröße (KG) der Sauerstoffsättigung aus der Anfangsphase (AP).

9. Verfahren nach einem der vorhergehenden Ansprüche, **gekennzeichnet durch** das Festlegen einer unteren Begrenzungslinie (60a) und einer oberen Begrenzungslinie (60b), wobei die untere Begrenzungslinie (60a) in der Hypoxiephase (HP) kleinere Werte der Kenngröße KG als die Referenzkurve (48) und die obere Begrenzungslinie (60b) größere Werte der Kenngröße (KG) als die Referenzkurve (48) zu jeweils gleichen Messzeiten aufweist, wobei die Begrenzungslinien (60a, 60b) insbesondere aus Messungen der Kenngröße (KG) an einer oder mehrerer Testpersonen bestimmt werden, wobei nur Werte der Kenngröße (KG) zur Bestimmung des Hypoxic-Session-Index (HSI) berücksichtigt werden, die kleiner als der Wert der Kenngröße (KG) in der oberen Begrenzungslinie (60b) und größer als der Wert der Kenngröße (KG) in der unteren Begrenzungslinie (60a) zu dem Zeitpunkt der Messung des jeweiligen Wertes der Kenngröße (KG) sind.

10. Verfahren nach einem der vorhergehenden Ansprüche, **gekennzeichnet durch** das Bestimmen des Hypoxic-Session-Index (HSI) mit Hilfe einer Veränderung einer Pulskurve (64) des Patienten während der Schritte III bis V.

11. Verfahren nach Anspruch 10, **gekennzeichnet durch** das Bestimmen eines Heartrate-Relaxation-Score (HRS) unter Auftragen einer Pulskurve (64), über die Zeit während des Hypoxietrainings, aufweisend die folgenden Schritte:
XXIV. Bestimmen des Mittelwerts des Pulses (RR) in der Anfangsphase (AP);
XXV. Auftragen des Mittelwerts des Pulses (RR) über die Dauer des Hypoxietrainings nach der Anfangsphase (AP) als Heartrate-Baseline (66) parallel zu der Zeitachse, wobei die Heartrate-Baseline (66) einen ersten Schenkel (68a) eines Relaxation-Messwinkels (RMW) bildet;
XXVI. Auftragen eines zweiten Schenkels (68b) des Relaxation-Messwinkels (RMW), wobei der zweite Schenkel (68b) durch den Puls (RR) am Anfangszeitpunkt (AZP) der Hypoxiephase (HP) und durch den Punkt (70) der Pulskurve (64) mit dem niedrigsten Wert des Pulses (RR) nach der Anfangsphase (AP) verläuft;
XXVII. Bestimmen des Heartrate-Relaxation-Score (HRS) als Maß des Relaxation-Messwinkels (RMW).

12. Verfahren nach einem der vorhergehenden Ansprüche, **gekennzeichnet durch** eine oder mehrere Wiederholungen der Schritte III bis V vor der Bestimmung des Hypoxic-Session-Index (HSI) gemäß Schritt VI.

## Claims

1. A method (100) of measuring a hypoxic session index (HSI) as a measure of the response of a patient to hypoxic training, with measurement of an index (KG) that characterizes the oxygen saturation in the patient's blood, having the steps:
I. Supplying the patient with a normoxic gas mixture (16) in an initial phase (AP) over a defined first period;
II. Determining the mean value of the index (KG) in the initial phase;
III. Supplying the patient with a hypoxic gas mixture (14) in a hypoxic phase (HP) over a defined hypoxic period;
IV. Supplying the patient with the normoxic or hyperoxic gas mixture (16) in a reoxygenation phase (RP) over a defined hyperoxic period and in a subsequent predetermined concluding period;
V. Plotting the index (KG) against time in the initial phase (AP), the hypoxic phase (HP) and the reoxygenation phase (RP) in the form of a data curve (46, 46a, 46b);
VI. Determining the hypoxic session index from a difference between the data curve (46, 46a, 46b) and a predetermined reference curve (48),
wherein the reference curve (48) is a data curve (46, 46a, 46b) which is determined for a reference person after steps I to V, or a data curve (46, 46a, 46b) which is calculated by averaging data curves (46, 46a, 46b) of a plurality of reference persons, wherein the data curves (46, 46a, 46b) are determined after steps I to V for the reference persons.

2. The method according to claim 1, **characterized by** setting a hypoxic cycle score (HCS) in the hypoxic phase (HP) for determining the hypoxic session index (HSI) by the following steps:
VII. Setting an HCS reference value (54) of the index (KG) that is smaller than the mean value of the index (KG) in the initial phase (AP) and is greater than a hypoxic safety value (52);
VIII. Plotting the HCS reference value (54) against time in the hypoxic phase (HP) as an HCS reference line (54) of the hypoxic phase (HP);
IX. Determining an HCS determination area (56) as an area between the data curve (46, 46a, 46b) and the HCS reference line (54) in the hypoxic phase (HP);
X. Determining an HCS reference area (58) as an area between the reference curve (48) and the HCS reference line (54) in the hypoxic phase (HP);
XI. Determining an HCS area difference between the HCS reference area (58) and the HCS determination area (56) and/or an HCS area ratio as the ratio of the HCS determination area (56) and the HCS reference area (58);
XII. Determining the value of the hypoxic cycle score (HCS) as a measure of the HCS area ratio and/or the HCS area difference.

3. The method according to any of claims 1 to 2, **characterized by** setting a reoxygenation max score (RMS) in the reoxygenation phase (RP) for determining the hypoxic session index (HSI) by the following steps:
XIII. Setting an RMS safety time (RSP) after the start of the reoxygenation phase (RP);
XIV. Determining the RMS safety value (RSW) of the index (KG) associated with the RMS safety time (RSP) on the data curve (46, 46a, 46b) in the reoxygenation phase (RP);
XV. Plotting the RMS safety value (RSW) against time (MT) in the reoxygenation phase (RP) as an RMS safety line (RSL) of the reoxygenation phase (RP);
XVI. Determining an RMS determination area (RBF₁, RBF₂) as an area between the data curve (46, 46a, 46b) and the RMS safety line (RSL) in the reoxygenation phase (RP);
XVII. Determining an RMS reference area (RRF) as an area between the reference curve (48) and the RMS safety line (RSL) in the reoxygenation phase (RP);
XVIII. Determining an RMS area difference between the RMS reference area (RRF) and the RMS determination area (RBF₁, RBF₂) and/or an RMS area ratio as the ratio of the RMS determination area (RBF₁, RBF₂) and the RMS reference area (RRF);
XIX. Determining the reoxygenation max score (RMS) as a measure of the RMS area ratio and/or the RMS area difference.

4. The method according to claim 3, **characterized by** determining a user reoxygenation potential (URP) as a measure of the difference between a predetermined reference reoxygenation max score (RMS) and the reoxygenation max score (RMS) determined in step XIX.

5. The method according to any of the preceding claims, **characterized by** setting a dynamic score (DS) in the hypoxic phase (HP) for determining the hypoxic session index (HSI), having the steps:
XX. Determining a DS reference time (DRZ) at which the index (KG) in the hypoxic phase (HP) has dropped to a defined DS reference value (DBW), wherein the DS reference value (DBW) is smaller than the mean value of the index (KG) in the initial phase (AP) and greater than the hypoxic safety value (52);
XXI. Determining the dynamic score (DS) as a measure of the time difference between the DS reference time (DRZ) and a defined DS reference time interval.

6. The method according to any of the preceding claims, **characterized by** setting a reoxygenation impulse score (RIS) in the reoxygenation phase (RP) for determining the hypoxic session index (HSI), having the steps:
XXII. Determining an RI reference time (RZP) to which the index (KG) in the reoxygenation phase (RP) has increased to a defined hyperoxic reference value (HB);
XXIII. Determining the reoxygenation impulse score (RIS) as a measure of the time difference between the RI reference time (RZP) and a defined RI reference time interval.

7. The method according to any of the preceding claims, **characterized by** setting an oxygen recovery score (ORS) in the reoxygenation phase (RP) for determining the hypoxic session index (HSI) by the reoxygenation impulse score (RIS) and the reoxygenation max score (RMS), in particular by averaging.

8. The method according to any of the preceding claims, **characterized by** determining a baseline potential (BP) as a measure of the difference between a predetermined ideal value of the index (KG) of the oxygen saturation and the mean value of the index (KG) of the oxygen saturation from the initial phase (AP).

9. The method according to any of the preceding claims, **characterized by** setting a lower boundary line (60a) and an upper boundary line (60b), wherein the lower boundary line (60a) in the hypoxic phase (HP) shows smaller values of the index KG than the reference curve (48), and the upper boundary line (60b) shows greater values of the index (KG) than the reference curve (48) at the respective same measurement times, wherein the boundary lines (60a, 60b) are determined, in particular, from measurements of the index (KG) in one or more subjects, wherein only values of the index (KG) are considered for determining the hypoxic session index (HSI), which values are smaller than the value of the index (KG) in the upper boundary line (60b) and greater than the value of the index (KG) in the lower boundary line (60a) at the time of measurement of the respective value of the index (KG).

10. The method according to any of the preceding claims, **characterized by** determining the hypoxic session index (HSI) by changing a pulse curve (64) of the patient during steps III to V.

11. The method according to claim 10, **characterized by** determining a heart rate relaxation score (HRS) by plotting a pulse curve (64) against time during hypoxic training, having the following steps:
XXIV. Determining the mean value of the pulse (RR) in the initial phase (AP);
XXV. Plotting the mean of the pulse (RR) against the duration of the hypoxic training after the initial phase (AP) as a heart rate baseline (66) parallel to the time axis, wherein the heart rate baseline (66) forms a first leg (68a) of a relaxation measurement angle (RMW);
XXVI. Plotting a second leg (68b) of the relaxation measurement angle (RMW), wherein the second leg (68b) runs through the pulse (RR) at the start time (AZP) of the hypoxic phase (HP) and through the point (70) of the pulse curve (64) having the lowest value of the pulse (RR) after the initial phase (AP);
XXVII. Determining the heart rate relaxation score (HRS) as a measure of the relaxation measurement angle (RMW).

12. The method according to any of the preceding claims, **characterized by** one or more repetitions of steps III to V before determining the hypoxic session index (HSI) according to step VI.

## Revendications

1. Procédé (100) dévolu à la mesure d'un indice de séance hypoxique (HSI) en tant que critère estimatif de la réaction d'un patient à un entraînement en hypoxie, avec mesure d'une grandeur caractéristique (KG) caractérisant la saturation en oxygène du sang du patient, comprenant les étapes consistant à :
I. délivrer, au patient, un mélange gazeux normoxique (16) au cours d'une phase initiale (AP), durant une première période préétablie ;
II. déterminer la valeur moyenne de ladite grandeur caractéristique (KG) au cours de ladite phase initiale ;
III. délivrer, au patient, un mélange gazeux hypoxique (14) au cours d'une phase d'hypoxie (HP), durant une période d'hypoxie préétablie ;
IV. délivrer, au patient, un mélange gazeux normoxique ou hypoxique (16) au cours d'une phase de réoxygénation (RP), durant une période d'hypoxie préétablie et durant une période d'achèvement prédéterminée consécutive ;
V. reporter, sous la forme d'une courbe de données (46, 46a, 46b), la grandeur caractéristique (KG) évoluant dans le temps au cours de ladite phase initiale (AP), de ladite phase d'hypoxie (HP) et de ladite phase de réoxygénation (RP) ;
VI. déterminer l'indice de séance hypoxique sur la base d'un écart de ladite courbe de données (46, 46a, 46b) vis-à-vis d'une courbe de référence (48) prédéterminée,
laquelle courbe de référence (48) est une courbe de données (46, 46a, 46b) déterminée chez une personne de référence d'après les étapes I à V, ou bien est une courbe de données (46, 46a, 46b) calculée en établissant une moyenne de courbes de données (46, 46a, 46b) de plusieurs personnes de référence, lesdites courbes de données (46, 46a, 46b) étant déterminées chez lesdites personnes de référence d'après lesdites étapes I à V.

2. Procédé selon la revendication 1, **caractérisé par** la spécification d'un score de cycle hypoxique (HCS) au cours de la phase d'hypoxie (HP), en vue de déterminer l'indice de séance hypoxique (HSI) par les étapes suivantes, consistant à :
VII. spécifier une valeur de référence HCS (54) de la grandeur caractéristique (KG) qui est inférieure à la valeur moyenne de ladite grandeur caractéristique (KG) au cours de la phase initiale (AP), et supérieure à une valeur hypoxique de sécurité (52) ;
VIII. reporter ladite valeur de référence HCS (54), évoluant dans le temps au cours de la phase d'hypoxie (HP), en tant que tracé de référence HCS (54) de ladite phase d'hypoxie (HP) ;
IX. déterminer une surface (56) de détermination HCS, en tant que surface entre la courbe de données (46, 46a, 46b) et ledit tracé de référence HCS (54) au cours de ladite phase d'hypoxie (HP) ;
X. déterminer une surface (58) de référence HCS, en tant que surface entre la courbe de référence (48) et ledit tracé de référence HCS (54) au cours de ladite phase d'hypoxie (HP) ;
XI. déterminer une différence de surfaces HCS, entre ladite surface (58) de référence HCS et ladite surface (56) de détermination HCS, et/ou un rapport de surfaces HCS en tant que rapport entre ladite surface (56) de détermination HCS et ladite surface (58) de référence HCS ;
XII. déterminer la valeur du score de cycle hypoxique (HCS) en tant que critère estimatif dudit rapport de surfaces HCS et/ou de ladite différence de surfaces HCS.

3. Procédé selon l'une des revendications 1 à 2, **caractérisé par** la spécification d'un score max de réoxygénation (RMS) au cours de la phase de réoxygénation (RP), en vue de déterminer l'indice de séance hypoxique (HSI) par les étapes suivantes, consistant à :
XIII. spécifier un point temporel (RSP) de sécurité RMS, après le début de la phase de réoxygénation (RP) ;
XIV. déterminer, sur la courbe de données (46, 46a, 46b), la valeur (RSW) de sécurité RMS de la grandeur caractéristique (KG) qui est associée audit point temporel (RSP) de sécurité RMS, au cours de ladite phase de réoxygénation (RP) ;
XV. reporter ladite valeur (RSW) de sécurité RMS, évoluant dans le temps (MT) au cours de ladite phase de réoxygénation (RP), en tant que tracé (RSL) de sécurité RMS de ladite phase de réoxygénation (RP) ;
XVI. déterminer une surface (RBF₁, RBF₂) de détermination RMS, en tant que surface entre la courbe de données (46, 46a, 46b) et ledit tracé (RSL) de sécurité RMS au cours de ladite phase de réoxygénation (RP) ;
XVII. déterminer une surface (RRF) de référence RMS, en tant que surface entre la courbe de référence (48) et ledit tracé (RSL) de sécurité RMS au cours de ladite phase de réoxygénation (RP) ;
XVIII. déterminer une différence de surfaces RMS, entre ladite surface (RRF) de référence RMS et ladite surface (RBF₁, RBF₂) de détermination RMS, et/ou un rapport de surfaces RMS en tant que rapport entre ladite surface (RBF₁, RBF₂) de détermination RMS et ladite surface (RRF) de référence RMS ;
XIX. déterminer le score max de réoxygénation (RMS) en tant que critère estimatif dudit rapport de surfaces RMS et/ou de ladite différence de surfaces RMS.

4. Procédé selon la revendication 3, **caractérisé par** la détermination d'un potentiel de réoxygénation d'utilisateur (URP) en tant que critère estimatif de la différence entre un score max prédéterminé de réoxygénation de référence (RMS) et le score max de réoxygénation (RMS) déterminé à l'étape XIX.

5. Procédé selon l'une des revendications précédentes, **caractérisé par** la spécification d'un score dynamique (DS) au cours de la phase d'hypoxie (HP), en vue de déterminer l'indice de séance hypoxique (HSI), comprenant les étapes consistant à :
XX. déterminer un point temporel (DRZ) de référence DS auquel la grandeur caractéristique (KG) a chuté jusqu'à une valeur de référence DS (DBW) préétablie, au cours de la phase d'hypoxie (HP), laquelle valeur de référence DS (DBW) est inférieure à la valeur moyenne de ladite grandeur caractéristique (KG) au cours de la phase initiale (AP), et supérieure à la valeur hypoxique de sécurité (52) ;
XXI. déterminer le score dynamique (DS) en tant que critère estimatif de la différence, dans le temps, entre ledit point temporel (DRZ) de référence DS et un intervalle de temps de référence DS préétabli.

6. Procédé selon l'une des revendications précédentes, **caractérisé par** la spécification d'un score d'impulsion de réoxygénation (RIS) au cours de la phase de réoxygénation (RP), en vue de déterminer l'indice de séance hypoxique (HSI), comprenant les étapes consistant à :
XXII. déterminer un point temporel (RZP) de référence RI auquel la grandeur caractéristique (KG) a cru jusqu'à une valeur d'hyperoxie de référence (HB) préétablie, au cours de la phase de réoxygénation (RP) ;
XXIII. déterminer le score d'impulsion de réoxygénation (RIS) en tant que critère estimatif de la différence, dans le temps, entre ledit point temporel (RZP) de référence RI et un intervalle de temps de référence RI préétabli.

7. Procédé selon l'une des revendications précédentes, **caractérisé par** la spécification d'un score de récupération d'oxygène (ORS) au cours de la phase de réoxygénation (RP), en vue de déterminer l'indice de séance hypoxique (HSI) par le biais du score d'impulsion de réoxygénation (RIS) et du score max de réoxygénation (RMS), notamment par établissement d'une moyenne.

8. Procédé selon l'une des revendications précédentes, **caractérisé par** la détermination d'un potentiel de base (BP) en tant que critère estimatif de la différence entre une valeur idéale prédéterminée de la grandeur caractéristique (KG) de la saturation en oxygène et la valeur moyenne de ladite grandeur caractéristique (KG) de la saturation en oxygène, fondée sur la phase initiale (AP).

9. Procédé selon l'une des revendications précédentes, **caractérisé par** la spécification d'un tracé inférieur de délimitation (60a) et d'un tracé supérieur de délimitation (60b), ledit tracé inférieur de délimitation (60a) présentant, au cours de la phase d'hypoxie (HP), des valeurs de la grandeur caractéristique KG moindres que la courbe de référence (48), et ledit tracé supérieur de délimitation (60b) présentant des valeurs de ladite grandeur caractéristique (KG) qui excèdent ladite courbe de référence (48), à des périodes de mesure respectivement identiques, sachant que lesdits tracés de délimitation (60a, 60b) sont notamment déterminés sur la base de mesures de la grandeur caractéristique (KG) effectuées sur une ou plusieurs personne(s) soumise(s) à des tests, seules étant alors prises en considération, pour déterminer l'indice de séance hypoxique (HSI), des valeurs de la grandeur caractéristique (KG) moindres que la valeur de ladite grandeur caractéristique (KG), sur le tracé supérieur de délimitation (60b), et excédant ladite valeur de la grandeur caractéristique (KG) sur le tracé inférieur de délimitation (60a) à l'instant de la mesure de la valeur respective de ladite grandeur caractéristique (KG).

10. Procédé selon l'une des revendications précédentes, **caractérisé par** la détermination de l'indice de séance hypoxique (HSI) à l'aide d'une modification d'une courbe (64) du pouls du patient au ours des étapes III à V.

11. Procédé selon la revendication 10, **caractérisé par** la détermination d'un score (HRS) de relaxation de la fréquence cardiaque, avec report d'une courbe du pouls (64) évoluant dans le temps au cours de l'entraînement en hypoxie, comprenant les étapes suivantes, consistant à :
XXIV. déterminer la valeur moyenne du pouls (RR) au cours de la phase initiale (AP) ;
XXV. reporter, parallèlement à l'axe du temps, ladite valeur moyenne du pouls (RR) pendant la durée de l'entraînement en hypoxie à l'issue de ladite phase initiale (AP), en tant que tracé de base (66) de la fréquence cardiaque, lequel tracé de base (66) de la fréquence cardiaque forme un premier côté (68a) d'un angle de mesure de relaxation (RMW) ;
XXVI. reporter un second côté (68b) dudit angle de mesure de relaxation (RMW), lequel second côté (68b) passe par le pouls (RR), à l'instant initial de la phase d'hypoxie (HP), et par le point (70) de la courbe du pouls (64) présentant la valeur la plus faible dudit pouls (RR) à l'issue de ladite phase initiale (AP) ;
XXVII. déterminer le score (HRS) de relaxation de la fréquence cardiaque en tant que critère estimatif dudit angle de mesure de relaxation (RMW).

12. Procédé selon l'une des revendications précédentes, **caractérisé par** une ou plusieurs réitération(s) des étapes III à V préalablement à la détermination de l'indice de séance hypoxique (HSI) conformément à l'étape VI.
